# EUROPEAN PATENT SPECIFICATION

(11) **EP 1 518 928 B1**
(45) Date of publication and mention of the grant of the patent: **18.02.2009**
(21) Application number: 04022696.1
(22) Date of filing: 23.09.2004
(51) Int. Cl.: C12N 15/11, A61K 31/7088, C12N 15/12, C07K 14/72

(54) **Antiandrogen oligonucleotides usable for the treatment of dermatological androgen-related disorders relating to androgen metabolism, their pharmaceutical compositions, their uses and treatment method**
Antiandrogen-Oligonukleotide zur Behandlung von Androgen-verwandten Hautkrankheiten, diese Verbindungen enthaltende pharmazeutische Zusammensetzungen und deren Verwendung
Oligonucléotides à activité antiandrogène pour le traitement des désordres cutanés associés aux androgènes. Compositions pharmaceutiques et leur utilisation

(30) Priority: 26.09.2003 AR 0303517
(43) Date of publication of application: 30.03.2005
(73) Proprietor: Fundacion Pablo Cassara, 1440 Buenos Aires (AR)
(72) Inventor: Kerner, Néstor Alberto, 1428 City of Buenos Aires (AR); Alvarez Roger, Maria Carolina, 1636 Olivos, Province of Buenos Aires (AR); Balana, Maria Eugenia, 1425 City of Buenos Aires (AR)
(74) Representative: Müller, Gerald Christian

(56) References cited:
- WO-A-01/83740
- WO-A-97/11170
- WO-A-03/017917
- US-A- 5 556 956
- US-A- 5 877 160
- US-B1- 6 489 163
- EDER I.E. ET AL.: "Inhibition of LNCaP prostate cancer cells by means of androgen receptor antisense oligonucleotides" CANCER GENE THERAPY, vol. 7, no. 7, 2000, pages 997-1007, XP002313666
- HAMY F. ET AL.: "Specific block of androgen receptor activity by antisense oligonucleotides" PROSTATE CANCER AND PROSTATIC DISEASES, vol. 6, 1 January 2003 (2003-01-01), pages 27-33, XP008041484
- BALANA M.E. ET AL.: "Antiandrogen oligonucleotides: active principles in hair- and skin-derived culture cells" JOURNAL OF DRUGS IN DERMATOLOGY, vol. 3, no. 3, May 2004 (2004-05), pages 287-294, XP008041485

### SEQUENCE LISTING

<110> Kerner, Néstor Alberto; Alvarez Roger, María Carolina; Balañá, María Eugenia
<120> Antiandrogen oligonucleotides usable for the treatment of dermatological androgen-related disorders relating to androgen metabolism, their pharmaceutical compositions, their uses and treatment method
<130> 62618
<140>
   <141>
<150> P03 01 03517
   <151> 2003-09-26
<160> 8
<170> PatentIn version 3.1
<210> 1
   <211> 18
   <212> DNA/RNA
   <213> artificial sequence
<223> oligonucleotide
<400> 1
   CATTGGTGAAGGATCGCC
<210> 2
   <211> 18
   <212> DNA/RNA
   <213> artificial sequence
<223> oligonucleotide
<400> 2
   CAATCATTTCTGCTGGCG
<210> 3
   <211> 20
   <212> DNA/R1VA
   <213> artificial sequence
<223> oligonucleotide
<400> 3
   GGCCTTCTTCGGCTGTGAAG
<210> 4
   <211> 20
   <212> DNA/RNA
   <213> artificial sequence
<223> oligonucleotide
<400> 4
   CACACGGTCCATACAACTGG
<210> 5
   <211> 18
   <212> DNA/RNA
   <213> artificial sequence
<223> oligonucleotide
<400> 5
   GGCGAAGTAGAGCATCCT
<210> 6
   <211> 18
   <212> DNA/RNA
   <213> artificial sequence
<223> oligonucleotide
<400> 6
   TGGCGCACAGGTACTTCT
<210> 7
   <211> 17
   <212> DNA/RNA
   <213> artificial sequence
<223> oligonucleotide
<400> 7
   CCACCACCACCACACGG
<210> 8
   <211> 18
   <212> DNA/RNA
   <213> artificial sequence
<223> oligonucleotide
<400> 8
   GCCGCCACCACCCCCACC

## Description

### FIELD OF THE INVENTION

The present invention relates to novel antiandrogen oligonucleotides suitable for the treatment of hair loss and androgen- metabolism related skin disorders, as well as relates to the corresponding topically administrable pharmaceutical and/or cosmetic compositions containing said oligonuleotides. The oligonucleotides according to the present invention are characterized by the following sequences:
INN-18.1 (SEQ ID N° 1): 5' CATTGGTGAAGGATCGCC 3'
INN-18.2 (SEQ ID N° 5): 5' GGCGAAGTAGAGCATCCT 3'

As a consequence of their molecular composition, these anti-androgenic active principles are very specific to reach their molecular target, which is especially circumscribed to the site of application, mainly the human androgen receptor in treated human skin and scalp. The oligonucleotides according to the present invention inhibit the androgen receptor (AR) expression at very low concentrations in skin and hair follicle primary cell cultures, through a mechanism implying the reaching of, and the hibridizing with, specific regions of the AR mRNA, thereby triggering the RNAse H digestion of the AR mRNA and thus inhibiting the AR translation.

The chemical structures for the two molecules INN-18.1 and INN-18.2 could be:
DNA (Deoxiribonucleic acid, phosphodiester bonds),
S-DNA (phosphorothioate bonds, {PTO}),
DNA and S-DNA mixed structure.

### DERIVED ALKYLATED OR METHYLATED CHEMICAL STRUCTURES, AS SUBSTITUENTS ON THE NITROGENOUS BASE.

### BACKGROUND OF THE INVENTION

### Biology of metabolic responses to androgen

Androgens mediate diverse metabolic responses through the androgen receptor (AR), a 110 kD nuclear receptor activated by specific ligands. The androgen receptor expression, which is found in a variety of tissues, can be activated by means of two ligands, testosterone and dihydrotestosterone, which bind with different affinities to the androgen receptor. A structural analysis of the AR indicated that the AR is a member of the steroid receptor superfamiliy, which includes the thyroid hormone receptor, the vitamin D receptor, and the retinoid receptor.¹²
¹ C. Chang, J. Kokontis & S. T. Liao: Structural analysis of complementary DNA and amino acid sequences of human and rat androgen receptors. Proc Natl Acad Sci USA 85 (1988), 7211-7215
² M. Beato: Gene regulation by steroid hormones. Cell 56 (1989), 335-344

The androgen receptor is a soluble protein. It is the result of a single gene located on the X chromosome which has been cloned ^{3 4 5 6}. This gene is responsible for the expression of, at least two, isoforms. The gene product is a protein including around 910 to 919 amino acids, with an N-terminal domain being more variable than other more preserved regions (DNA binding domain, nuclear location region, ligand binding domain)⁷ with a Region of poly CAG ( polyglutamyl region ) sequences having variable lengths and apparently involved in the appearance of diverse pathologies associated with the chromosome X. ⁸
³ Chang C., J. Kokontis & S. Liao, Science 240 (1988), 324-326 ⁴ Lubahn D., Brown T., Simenthal et al, PNAS 86 (1988), 9534-9538
⁵ Chang C., J. Kokontis & S. Liao, PNAS 85 (1988), 7211-7215
⁶ Tilley W., M. Marcelli, J. Wilson & M McPhaul, PNAS 86 (1989), 327-331
⁷ Chang C., C. Wang, H. DeLuca, T. Ross & C. Shih, PNAS 89 (1992), 5946-5950
⁸ LaSpada A., E. Wilson .,D. Lubahn, A. Harding & K Fischbeck,Nature 352 (1991), 77-79

A B C D E

H2N-                                                                                    -- COOH

1    20                                        559    624  676                        919

A, B, C: transcriptional activation region (B: polyglutamyl region)

D: DNA binding region

E: ligand binding region

The AR expression is getting changed through development and aging, as well as through a malignant transformation in the course of several oncogenic processes. The androgen receptor acts as a transcriptional modifier over the transcriptional activity of a variety of genes, through the binding to an androgen responsive element (ARE) as well as through the interaction with other cell specific transcriptional factors and certain DNA elements acting in "cis" next to the ARE. The biological activity released by the androgen receptor varies with different cell targets and even inside a single tissue type.

The androgen receptor expresses itself in high levels within several kinds of reproductive cells, playing an important role in the development and maintenance of sex functions; it also expresses in non - reproductive tissues, regulating a number of enzymes and proteins. However, there is a belief that an abnormal regulation of the androgen receptor gene is a significant cause for hormone - dependent disorders.

On the basis of the observations that androgens play a role in the development of hormone-dependent disorders, the actions of certain steroidal and non - steroidal anti-androgens have been investigated in order to treat them.

Among the disorders having a social impact, outstanding are prostate cancer, skin disorders of the androgen - dependent kind and other hormone-dependent pathologies such as Benign Prostatic Hyperplasia (BPH). BPH is a common benign growth in aging men. This non-cancerous enlargement of the prostate gland frequently causes symptoms in the lower urinary tract. It is a progressive disease which, if untreated for a reasonable period of time, can give rise to a reduction in the patient's quality of life.

In males, the androgen receptor within the hair follicle may be responsible for cutting out growth and consequently lead to baldness, as well as, paradoxically, said receptor may induce hair growth in other kind of follicles.

Biology of hair growth thus offers an example of the different effects that androgens may exert on the proliferation of similar populations of epithelial cells. Specifically, on one hand, testosterone and its metabolite, dihydrotestosterone (DHT), can stimulate facial hair growth, while on the other hand, they cause the regression of hair follicles in aging individuals.⁹ Hair follicles are intimately associated with the mesenchymally-derived dermal papilla, which is believed to have a substantial influence on follicular proliferation.
⁹ V. A. Randall: Androgens and human growth. Clin. Eondocrinol 40, 439-57 (1994)

Different lines of evidence support the role of androgens in controlling growth of hair follicles through modulation of the dermal papilla activity:
1) The AR has been identified in the dermal papilla. ^{10 11}
   ¹⁰M. B. Hodgins, R. Choudhry, G. Parker, R. F. Oliver, C. A. Jahoda, A. P. Withers, A. O. Brinkmann, T. H. van der Kwast, W. J. Boersma, K. M. Lammers & a. et: Androgen receptors in dermal papilla cells of scalp hair follicles in male pattern baldness. Annals New York Acad Sci 642, 448-51 (1991)
   ¹¹ R. Choudhry, M. B. Hodgins, T. H. Van der Kwast, A. O. Brinkmann & W. J. Boersma: Localization of androgen receptors in human skin by immunohistochemistry: implications for the hormonal regulation of hair growth, sebaceous glands and sweat glands. J Endocrinol 133, 467-75 (1992
2) Dermal papillae from androgen-dependent hair follicles seem to contain a greater number of AR than those in areas not predisposed to originate baldness. ¹²
   ¹² V. A. Randall, M. J. Thornton & A. G. Messenger: Cultured dermal papilla cells from androgen-dependent human hair follicles (e.g. beard) contain more androgen receptors than those from non-balding areas of scalp. J Endocrinol 133, 141-7 (1992
3) The level of 5α-reductase, enzyme responsible for the transformations of testosterone into dihydrotestosterone, varies among the hair follicles existing within the frontal and the occipital zones¹³.
   ¹³ M. E. Sawaya: Biochemical mechanisms regulating human hair growth. Skin Pharmacol 7, 5-7 (1994)
4) Dermal papillae can produce extracellular matrix components and mitogenic factors⁷.
   ⁷ Chang C., C. Wang, H. DeLuca, T. Ross & C. Shih, PNAS 89 (1992), 5946-5950

The dermal papilla (DP) is in charge of directing the embryonic generation of hair follicles and retains this instructive ability throughout the life of the follicle. The DP is composed of a small group of fibroblastic cells derived from the mesoderm. These cells are held near the base of epidermal cells that produce hair fiber and root sheaths. The DP has the shape of a perfect "pear" in normal hair follicles. These are a highly active group of cells able to inducing follicle development from the epidermis and producing hair fiber ^{14 15} . ¹⁶
¹⁴ Oliver RF, Regeneration of dermal papillae in rat vibrissae.J Invest Dermatol. 1966;47:496-497.
¹⁵ Oliver RF, Histological studies of whisker regeneration in the hooded rat.J Embryol Exp Morphol. 1966 16:231-244.
¹⁶ Oliver RF, The experimental induction of whisker growth in the hooded rat by implantation of dermal papillae. J Embryol Exp Morphol. 1967, 18: 43-51.

All these findings suggest that androgens, via the AR, can indirectly mediate an effect on hair follicle proliferation, through the modulation of dermal papillae activity. The effect of androgens on cell proliferation in hair follicles may be controlled through the expression regulation of growth factors.

Androgens can gradually diminish the size of scalp hair follicles until baldness takes place. Although the exact mechanism through which baldnes occurs, it has been shown that there is a direct correlation between androgen levels and hair loss. A diminution of androgen content should lead to a reduction of baldness and a re-growth of hair.

Androgenetic alopecia is a very common form of hair loss and could be described as part of a general genetic phenotype. Alopecia is mediated by systemic androgens and genetic factors. It is characterized by a progressive hair loss, especially in scalp established regions, It develops as a gradual reduction of scalp hair follicle size, and it shortens the time in the active growth phase (anagen), this leading to more hair follicles in the resting stage (telogen) of the follicle cycle. In men, the hair loss takes place on the top of the head and can involve hair thinning and hair line receding.

Androgenetic alopecia is a specific type of hair loss characterised by progressive, patterned hair loss from the scalp, mediated by systemic androgens and genetic factors. Androgenetic alopecia is a consequence of a genetic predisposition and sufficient circulating androgens.

Every white man possesses the autosomal inherited predisposition. Recent advances in understanding of the biology of hair follicles have shed light on the pathogenesis of androgenetic alopecia. Androgenetic alopecia affects between 50 and 80% of Caucasian male population. As a rule, it affects around 30% of men in their thirties. At 40, alopecia appears in 40%, and so on progressively until getting to be 80% of men as from 80 years of age. Different ethnic backgrounds have different susceptibility levels as regards the development of androgenetic alopecia. Hair loss starts only after puberty, and the progression rate is significantly variable: some men go completely bald in less than 5 years, while most take from 15 to 25 years. A study showed that the average rate of hair loss of about 5% per year. Progression fluctuates considerably, with periods of accelerated loss lasting from 3 to 6 months, followed by quiescent periods lasting from 6 to18 months.

Despite its standard name of "male pattern of hair loss" androgenetic alopecia is also the most common form of hair loss in women.

Terminal anagen hairs, which normally penetrate through the dermis into the subcutis, are replaced by secondary vellus hairs with residual angiofibrotic tracts. An additional feature is an increased ratio of telogen to anagen hairs. The total number of hair follicles for an adult human is estimated at 5 million with 1 million on the head, of which 100,000 cover the scalp. The basic hair follicle structure is essentially the same throughout the whole range of mammalian species, with modifications for some specialized functions. The hair follicle can be recognized as a separate organ within the skin, being formed and kept on the basis of an interaction between dermal and epidermal components.

On the other hand, the disorder known as acne vulgaris is a chronic inflammatory condition of the pilosebaceous units. Although acne is not generally associated with high serum levels of androgens, it has been shown that female acne patients definitely exhibit increases in ovarian and adrenal androgen levels.

### ANTI-ANDROGENIC THERAPIES

A number of preparations for treatment and prevention of hair loss that are well known and used for a long time now. has been formulated: for example, preparations based on biological products, vascular toners and vasodilators, testosterone blockers and even the practice of performing surgical hair transplants.

Anti-androgens antagonize the biological responses induced by endogenous or exogenous androgens. Their methods of action vary from drug to drug. The specificity of androgen action is accomplished through both the specific recognition of target genes, as well as through the specificity of the androgenic hormone interaction with the AR¹⁷
¹⁷ Raynaud JP, Ojasso T, The design and use of sex-steroid antagonists. J Steroid Biochem (1986), 25:811-833

Steroidal anti-androgens, such as cyproterone acetate (Androcure ^{™}) act by blocking the binding of hormone ( testosterone or DHT) to the AR, as well as by exerting progestational effects which suppress gonadotropin secretion. The side effects because of the use of these anti-androgens could be the cause of male feminization or a potential teratogenicity.

Non-steroidal anti-androgens do not have the steroidal's side effects:
they exist as pure anti-androgens that bind exclusively to the AR. However, due to side effects, some of those anti-androgens, such as bicalutamide (Casodex®), are exclusively indicated for prostate cancer treatment, a general use being prevented for other hormone-dependent disorders. Nowadays, for the treatment of diverse pathologies related to androgens, drugs such as flutamide (Eulexin ^{™}), hydroxiflutamide or nilutamide (Anandron ^{™}) are being employed.

Combination therapies use an anti-androgen with a 5 α-reductase inhibitor such as finasteride (Proscar ^{™}). Within certain sensitive hormone tissues (prostate, certain skin regions), the circulating testosterone is converted into the more potent DHT by the enzyme 5 α-reductase

Dutasteride is a drug similar to Finasteride, which prevents the hormone Testosterone from being converted into DihydroTestosterone (DHT). Dutasteride is a 5-alpha reductase inhibitor that inhibits both the type 1 and 2 isoenzymes. Nowadays, this drug, developed by GIaxoSmithKline, is going through the final stages of the approval process for its use for Benign Prostatic Hyperplasia (BPH). Studies concerning its employment for the treatment of hair loss seem to be stopped.

In view of the populational penetration and the importance of these pathologies, an increased use of anti-androgens is expected, because of an aging population, a growing number of patients suffering from BPH, and an increased awareness of the condition and a trend towards preventing long term complications.

Minoxidil ("Rogaine" ^{™}) is a powerful vasodilator that is being used in the medical treatment of baldness. Because of the scarce and variable efficacy and of its side effects, minoxidil quickly became a disappointment. Recently, to the market has been launched finasteride, formulated at 1 mg (Propecia " ^{™}) as a 5-alpha-reductase inhibitor which reduces circulating levels of the dihydrtestosterone (DHT) produced by testosterone. Since the strategy is to reduce the levels of systemic DHT finasteride must be administered orally because a topical administration has a very low efficiency.

Current acne treatments include oral contraceptives plus a complement of antibiotics, which can be administrated topically or systemically. The estrogen-progestin combination with cyproterone acetate (CPA) has a direct peripheral anti-androgenic action on the blocking of the androgen receptor.

Spironolactone has been used for over 20 years now as an antiandrogen for the treatment of acne and hirsutism. However, only a few studies on a small number of patients have shown some efficacy of spironolactone used as sole therapy or combined with oral contraceptives for the treatment of acne. Side effects are of common occurrence, but they are not a usual cause for stopping the treatment.

### OLIGONUCLEOTIDES AS PHARMACOLOGICAL AGENTS

Progress in the fields of biology, genetics and genomics has enabled the study of some molecular mechanisms that regulate the expression of genes which could be the bases for the triggering of diverse pathologies. From this data it has become clear the need to find out pharmaceuticals capable of precluding, in an extremely specific way, the expression of those genes involved. That is why oligonucleotides which may act in different ways have been selected as a new therapeutic class. These oligonucleotides selectively interfere with, or prevent, the expression of specific proteins, thus offering a means of intervening in fundamental processes that cause certain diseases. They could act through different routes, such as, for instance, the promotion of the mRNA specific degradation via an RNAse H digestion, a translation blocking, as ribozymes, as small interferent RNA's (RNAi), or as aptameres on DNA regulating proteins. The secondary effects on normal processes are minimal.

The main way of action of pharmacologically active oligonucleotides is their capability of binding to their molecular metabolic targets. According to this objective, oligonucleotides should be designed such that they can reach the accessibility regions in the target molecule as , for instance, in the mRNA.

There exist several theoretical models that predict the secondary and tertiary molecular structures and focus on the possibility of revealing accessibility regions. Concerning the RNA it has been developed a software MFOLD¹⁸ and, more recently, a bioinformatic algorithm¹⁹ which should be useful to identify single strand short sequences. However, the results obtained are, in general, disappointing, when they are confronted to the tested ones in biochemical assays on binding to messenger RNA, especially when the size of the mRNA is larger than 800 oligonucleotides.
¹⁸ M. Zucker, On finding all suboptimal foldings of an RNA molecule. Science 244, (1989), 48 - 52
¹⁹ Y. Shigenobu & Del Carpio C., A Bioinformatic Approach for RNA 3D Structure Prediction: Development of a Knowledge-Base for 2D-to-3D Structural Elements Compatibility Analysis, Genome Informatics 12 (2001).

Oligonucleotides are already on the market, after the approval of fomivirsen (Vitravene ^{™}), for the treatment of cytomegalovirus retinitis. Besides, other products are to be launched soon, following the encouraging results of clinical trials carried out on five therapeutic areas: cancer, immune system-related disorders (inflammatory disorders, transplant rejection, vaccine adjuvants), cardiovascular (vascular restenosis, hypertension), neurological disorders (neurodegenerative, cerebrovascular disorders, neurotransmitter regulation) and infectious diseases (antiviral infections, anti protozoal parasites).

Roy et al.20 20 (US Patent 5,556,956) disclosed methods and compositions including antisense and antigene constructs, to the purpose of being used in the regulation of the expression of the androgen receptor. Roy et al.'s strategy is interfering with the AR expression by using oligonucleotides capable of inhibiting the transcription by means of a triple helix formation, aiming to the promoter region of the androgen receptor (AR) gene.
²⁰ US-5,556,956

Harper et al.21 (US Patent 5,877,160) described a method of decreasing androgen-associated hair loss, by exposing scalp cells to an effective amount of oligonucleotides which interact with coding regions of the AR gene, with its transcription or with a target sequence on direction 3' of the transcription start site of said gene. In particular, Harper's olignucleotides target the position from -500 to +20 relative to the transcription start site. However, his results do not show a pharmacological activity in cell types involved in the hair folicle cycle and, consequently, involved in human hair growth.
²¹ US-5,877,160

F. Hamy et al., Prostate Cancer and Prostatic Diseases (2003), 6, 27-33, and Iris E. Eder, Cancer Gene Therapy, Vol 7, No 7, 2000: pp 997-1007, reported about antiandrogen receptor antisense oligonucleotides and their application in therapy of prostate cancer.

Recently, therapeutic compositions and methods for inhibiting expression of full length proteins in cells and their application in therapy of prostate cancer, and in particular to antisense compositions targeted against an mRNA sequence of a normal splice acceptor junction in a preprocessed mRNA have been reported (WO 01/83740).

### HIGH SPECIFICITY ANTI-ANDROGEN THERAPY

Androgenetic alopecia and androgen-related skin disorders still need to be approached by a very specific, safer and less toxic anti-androgen therapy capable of being applied in a localized way, so as to avoid the negative consequences and secondary effects of a more general systemic anti-androgen therapy.

The main problem to be solved is the generation of new anti-androgen drugs to be especially applied through a topical route, without the risk of reaching other non-implicated potential organs.

The oligonucleotides targeting the androgen receptor seem to be the most appropriate pharmacological approach. The pharmacological activity of those oligonucleotides should be previously demonstrated in cell models properly established, in primary cultures of skin fibroblasts and in dermal papilla cells from human hair follicles. The concentration range within which the activity and the inhibition levels achievable in those models must be assessed. Thus defined, these oligonucleotides seem to be apt to study an anti-androgen therapy, after a prior definition of a galenic formulation adequate to a topic administration.

### SUMMARY OF THE INVENTION

Therefore, it is an object of the invention to provide pharmacologically active oligonucleotides having an anti-androgen activity and acting specifically upon the desired target.

It is another object of this invention to provide pharmacologically active oligonucleotides that join highly sensitive regions in the androgen receptor mRNA.

More particularly, another object of the invention is to provide pharmacologically active nucleotides that inhibit the androgen receptor expression in human skin fibroblasts and dermal papillae cells derived from hair follicles.

More specifically, another object of the invention is to provide pharmacologically active oligonucleotides that inhibit in a specific way the androgen receptor expression at very low concentrations, without causing the side effects created by the anti - androgen therapies from the previous art.

More specifically, another object of this invention is to provide pharmacologically active oligonucleotides that specifically inhibit androgen - related hair loss and skin conditions.

Another object of the invention is to provide a pharmaceutical composition including pharmacologically active oligonucleotides that specifically inhibit the androgen receptor expression at very low concentrations.

Another object of the invention is to provide a pharmaceutical composition including pharmacologically active oligonucleotides having an anti - androgen activity for a topic administration.

More specifically, another object of the invention is to provide a pharmaceutical composition comprising pharmacologically active oligonucleotides having an anti - androgen activity, in the form of an aqueous solution.

Another object of the invention is the use of pharmacologically active oligonucleotides to make a pharmaceutical composition for the treatment of hair loss and skin conditions androgen - related.

A last object of the invention is a method for the treatment of androgen - related hair loss in human beings.

### BRIEF DESCRIPTION OF FIGURES

Figure 1 shows an autoradiography of the electrophoretic mobility shift analysis (EMSA) of radiolabeled inn - 18.1 (+³² p - INN 18.1), hybridized separately to AR sense and antisense transcripts of AR.
Figure 2 shows an autoradiography of the electrophoretic mobility shift analysis (EMSA) of INN - 18.1, showing the effect of the competition during the hybridization using combinations of INN-18.1 labelled with increasing concentrations of unlabeled INN-18.1 (lanes 2-4) and in combination with increasing concentrations of non - related and unlabeled (control) oligonucleotide.
Figure 3 shows the results of denaturing polyacrylamide gel electrophoresis of the assay of the protection against S1 Nuclease degradation, by INN-18.1 oligonucleotide.
Figure 4 shows the results of agarose denaturing gel electrophoresis for the RNAase H digestion induced by the INN-18.1
Figure 5 shows the results of SDS polyacrylamide gel electrophoresis of in vitro translated AR after the treatment with the INN-18.1 oligonucleotide in the two step process.
Figure 6 shows the immunoblotting analysis for the AR expression inhibition, after the treatment of primary cultures of dermal fibroblasts with diverse concentrations of INN-18.1.
Figure 7 shows the immunoblotting analysis of the AR expression inhibition after the treatment of primary cultures of dermal papilla cells with diverse concentrations of INN-18.1.
Figure 8 shows an autoradiography of the electrophoretic mobility shift analysis (EMSA) of radiolabeled INN - 24 (+³² P - INN 24), separately hybridized separately to sense and antisense transcripts of AR.
Figure 9 shows an autoradiography of the electrophoretic mobility shift analysis (EMSA) of INN - 24, showing the effect of the competition during the hybridization using combinations of INN-24 labeled with increasing concentrations of unlabeled INN-24 (lanes 2-4) and in combination with increasing concentrations of non - related and unlabeled (control) oligonucleotide.
Figure 10 shows the results of denaturing poliacrylamide gel electrophoresis analysis of the assay of the protection against S1 Nuclease degradation, by INN-24 oligonucleotide.
Figure 11 shows the results of agarose denaturing gel electrophoresis for the RNAse H digestion induced by the INN-24.
Figure 12 shows the results of SDS polyacrylamide gel electrophoresis of in vitro translated AR after the treatment with the INN-24 oligonucleotide in the two step process.
Figure 13 shows the immunoblotting analysis for the AR expression inhibition, after the treatment of primary cultures of dermal fibroblasts with diverse concentrations of INN-24.
Figure 14 shows the immunoblotting analysis of the AR expression inhibition after the treatment of primary cultures of dermal papilla cells with diverse concentrations of INN-24.
Figure 15 shows an autoradiography of the electrophoretic mobility shift analysis (EMSA) of radiolabeled INN - 71 (+³² P - INN 71), separately hybridized to sense and antisense transcripts of AR.
Figure 16 shows an autoradiography of the electrophoretic mobility shift analysis (EMSA) of INN - 71, showing the effect of the competition during the hybridization using combinations of INN-71 labeled with increasing concentrations of unlabeled INN-71 (lanes 2-4) and in combination with increasing concentrations of non - related and unlabeled (control) oligonucleotide.
Figure 17 shows the results of denaturing poliacrylamide gel electrophoresis analysis of the assay of the protection against S1 Nuclease degradation, by INN-71 oligonucleotide.
Figure 18 shows the results of agarose denaturing gel electrophoresis for the RNAse H digestion induced by the INN-71.
Figure 19 shows the results of SDS polyacrylamide gel electrophoresis of - in vitro translated AR after the treatment with the INN-71 oligonucleotide in the two step process.
Figure 20 shows the immunoblotting analysis for the AR expression inhibition, after the treatment of primary cultures of dermal fibroblasts with diverse concentrations of INN-71.
Figure 21 shows the immunoblotting analysis of the AR expression inhibition after the treatment of primary cultures of dermal papilla cells with diverse concentrations of INN-71.
Figure 22 shows the immunoblotting analysis of the AR expression inhibition after the treatment with primary cultures of dermal papilla cells with diverse concentrations of INN-18.2.
Figure 23 shows the immunoblotting analysis of the AR expression inhibition after the treatment of primary cultures of dermal papilla cells with diverse concentrations of INN-24.1.
Figure 24 shows the immunoblotting analysis of the AR expression inhibition of primary cultures of dermal papilla cells with diverse concentrations of INN-72.
Figure 25 shows the immunoblotting analysis of the AR expression inhibition of primary cultures of dermal papilla cells with diverse concentrations of INN-73.
Figure 26 shows the immunoblotting analysis of the AR expression inhibition of primary cultures of dermal papilla cells with diverse concentrations.

### DETAILED DESCRIPTION OF THE INVENTION

It has been surprisingly found that androgen receptor expression can be modulated by the following oligonucleotides:
INN-18.1 (SEQ 10 N° 1): 5' CATTGGTGAAGGATCGCC 3'
INN-18.2 (SEQ ID N° 5): 5' GGCGAAGTAGAGCATCCT 3'

The design of these active principles is the consequence of having been found new accessibility regions on the Androgen Receptor (AR) mRNA 3-D structure. These new accessibility regions in turn allow the oligonucleotides called INN-18.1 and INN-18.2 (ODN) to inhibit the AR expression at very low concentrations (Nanomolar concentrations) in human skin derived and hair follicle primary cell cultures, by targeting specific regions of the mRNA of the AR:
INN-18.1 (SEQ ID N°1) +2251
INN-18.2 (SEQ ID N° 5) + 2277
   (position of nucleotides on a mRNA molecule, considering as nucleotide +1 the translation start point).

Thus, the RNAse H digestion of the AR mRNA is triggered and a significant diminution of the AR expression levels (an inhibition rate between 60%-80%, approximately) is produced.

According to the present invention, the oligonucleotides whose sequences are presented intend to encompass both DNA (deoxiribonucleotide acid, phosphodiester bonds), S-DNA (phosphorothioate, {PTO}, bonds), DNA/S-DNA (PTO) mixed structures or alkylated or methylated chemical structures derived, as substituents on the nitrogene base.

These antiandrogen oligonucleotides are chemically synthesized and after penetrating into the cell they act pharmacologically thanks to their capacity to trigger the degradation of the targeted mRNA. It happens to be a mechanism similar to ARN degradation in the intracellular heteroduplex RNA / DNA naturally taking place by RNAase H.

In order to design anti - androgen molecules pharmacologically active in hair follicles and skin derived cells, an approach comprised of two - parts was carried out:
i. In vitro assesment, with the purpose of disclosing accessibility regions in the AR mRNA.
ii. Anti - androgen activity in cell models.

### i. In vitro assesment, with the purpose of disclosing accessibility regions in the AR mRNA.

The rational of this procedure is the discovery of accessibility regions in the mRNA molecule of the AR, through the binding ("hybridization") of oligonucleotides the sequence of which are complementary to those of the examined region. Therefore, when an accessibility region is discovered, specific sequence oligonucleotides (ODN) targetting that region are designed, thus it can be established whether they attain a thorough hybridization.

To design active principles, the strategy called "walking on the mRNA" was adapted. It makes possible the in vitro identification of accessibility sites in the AR-mRNA appropriate for hybridization of the oligonucleotides. It is based on targeting regions spread out on the mRNA molecule, select sections 15 and 20 nucleotides long, where the putative cross matching with any other expressed gene of the human genome is non significant, and then moving on sequences in the direction 3' and 5'. Once an accessibility region is disclosed, the ODN covering the surrounding sequences are designed and then tested so as to disclose a pharmacological activity.

The "walking on the mRNA strategy" is the adapted version of the " walking on the gene strategy" which is used to achieve the sequencing of long pieces of DNA, starting from targeting a known region and then moving in directions 3' and 5' from the newly sequenced regions and so on. It is therefore possible "to walk" on the DNA, in order to disclose the surrounding sequences. This strategy was modified so as to assess the binding capability of the oligonucleotides on the mRNA, thus disclosing new accessibility regions "walking" on the mRNA molecule or else on those pharmacologically active, to find new anti-androgen oligonucleotides through walking within the accessibility regions detected.

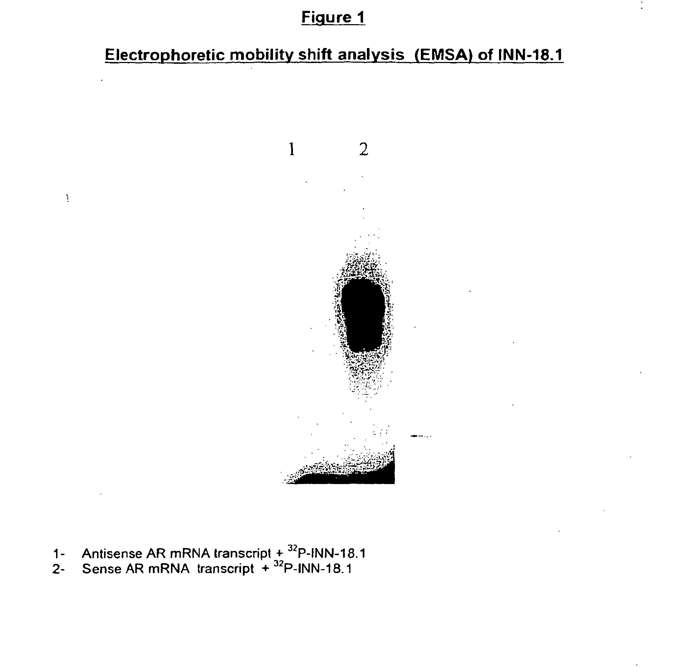

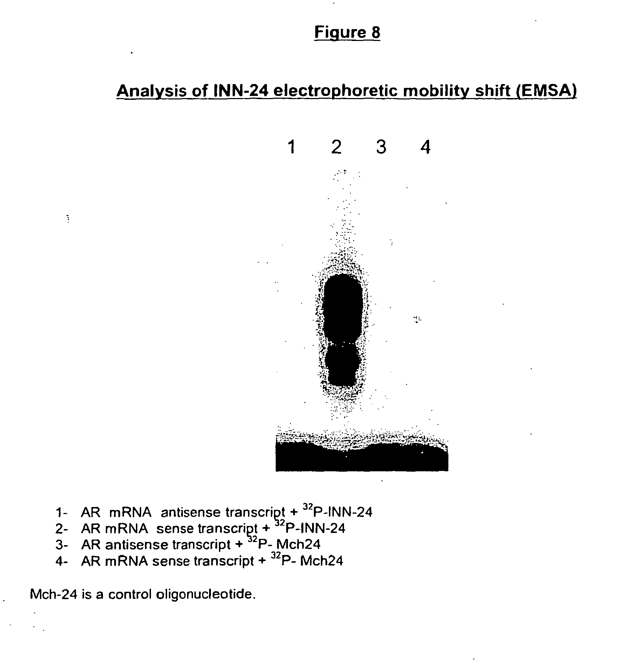

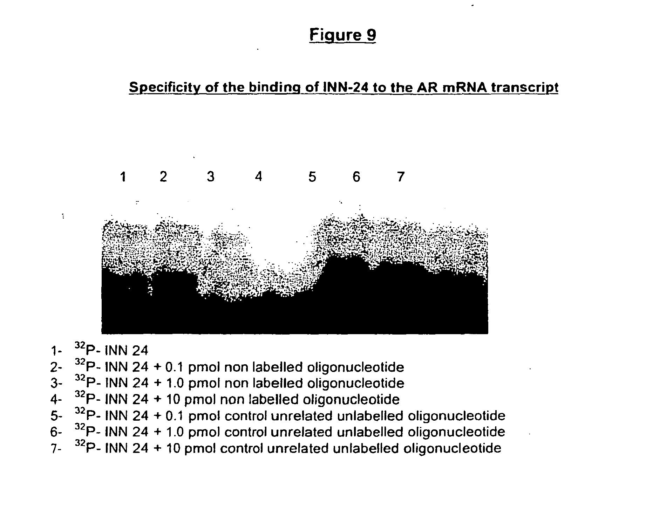

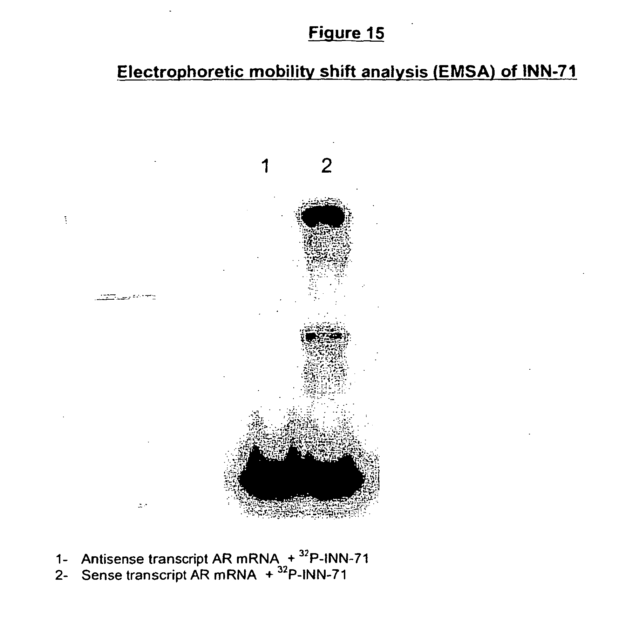

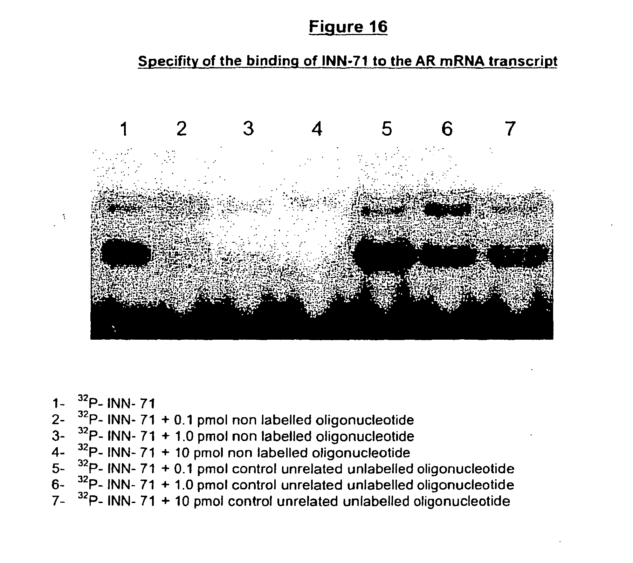

According to this walking strategy, on the human androgen receptor mRNA was tested whether oligonucleotides targeting different regions of the human AR mRNA were capable of binding to the mRNA molecule. Therefore the mRNA molecule was divided into seven regions (1 to 7) containing about 600 nucleotides each. Afterwards, 3 oligonucleotides were designed that targeted each region and for which the sequence homology with any human gene is non significant, to thus test whether they were capable of binding to the mRNA molecule through the *Electrophoretic Mobility Shift Analysis,* EMSA (see figures 1, 2, 8, 9, 15, 16).

Each oligonucleotide has been designed as a complement to the region established as a target in the mRNA. The design of other oligonucleotides to tested by means of EMSA was performed moving the sequence 5 nucleotide in direction 3' and in direction 5', following the surrounding mRNA sequence established as a target by the oligonucleotide previously designed. The *Electrophoretic Mobility Shift* Analysis (EMSA) indicates whether the oligonucleotide could bind, or not, to the AR mRNA, to achieve the hybridization, thus an accessibility region is being disclosed. This technique consists of the following steps: a) Oligonucleotide radio-labeling and purification; b) In vitro transcription of Androgen Receptor (AR) mRNA; c) Hybridization of AR mRNA transcript with radio-labeled oligonucleotides; and d) Casting of non-denaturing gel, electrophoresis and autoradiography.

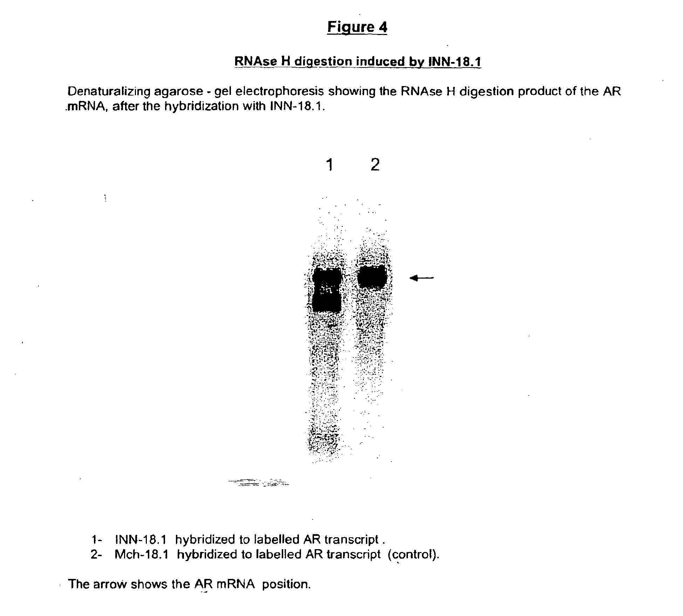

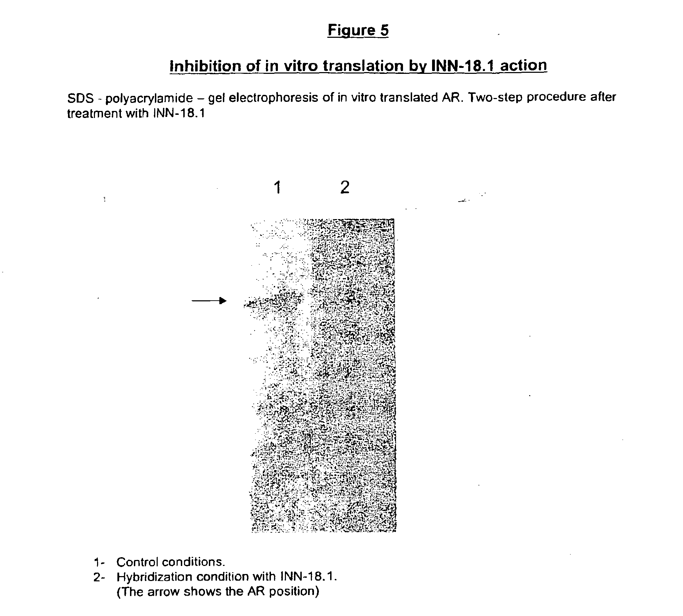

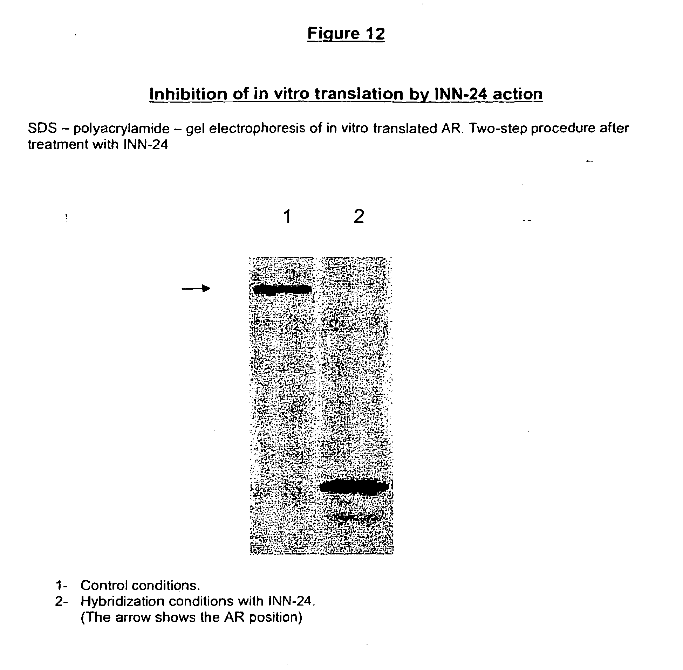

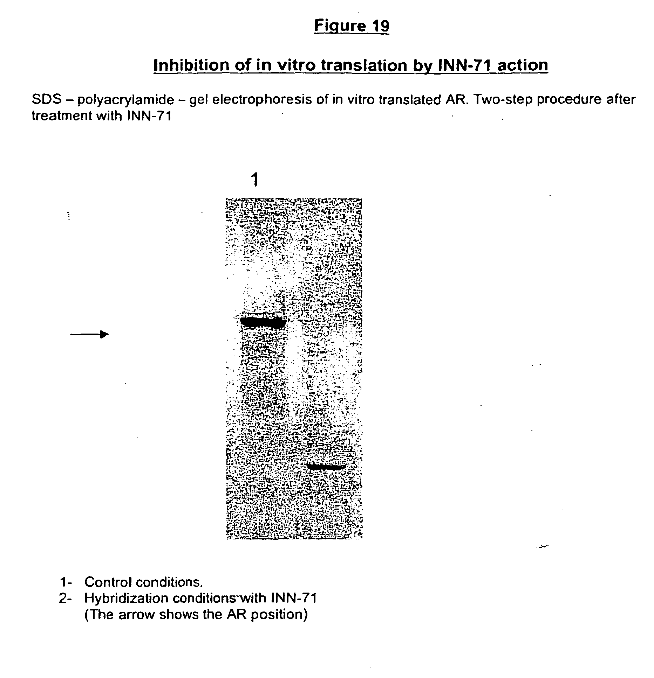

As a consequence of this first part of the work, groups of positive oligonucleotides were established.
**1) Oligonucleotides Positive for EMSA:** those capable of binding to the mRNA molecule.
   The oligonucleotides that did not manage to bind specifically to the mRNA were discarded.
   EMSA - Positive oligonucleotides bound to the mRNA molecule were tested in the nuclease S1 protection assay, that indicates whether the oligonucleotide hybridization was partial or complete. If a degradation does not take place, hybridization was complete, keeping the oligonucleotide the length after digestion. Thus the second group of nucleotides is established.
**2) Oligonucleotides positive for the protection of S1 nuclease digestion:**
   Were taken into consideration those whose degradation was prevented and that kept complete the complementary sequence.
   The oligonucleotides that lost size as a consequence of this test were discarded. Positive oligonucleotides were considered apt to be tested for RNAse H digestion, what shows that the ODN's hybridization capacity is able to trigger a mechanism of mRNA digestion by RNAse H which, within cells, will be responsible for the AR-mRNA degradation. Thus the third group of nucleotides was established.
**3) Oligonucleotides positive for the mRNA digestion with RNAse H:** those capable of triggering the mRNA degradation were considered (see figures 4, 22, 28)
   Afterwards, these positive oligonucleotides are tested based on their capability for triggering a mRNA degradation via the RNAse H mechanism and thus inhibiting the in vitro expression of the androgen receptor (AR).
**4) Oligonucleotides positive for the in vitro translation inhibition:** those capable of inhibiting the expression of the androgen receptor molecule, as a consequence of the RNA degradation triggered by RNAse H mechanism (see figures 5, 12, 19)

### Mechanism of action

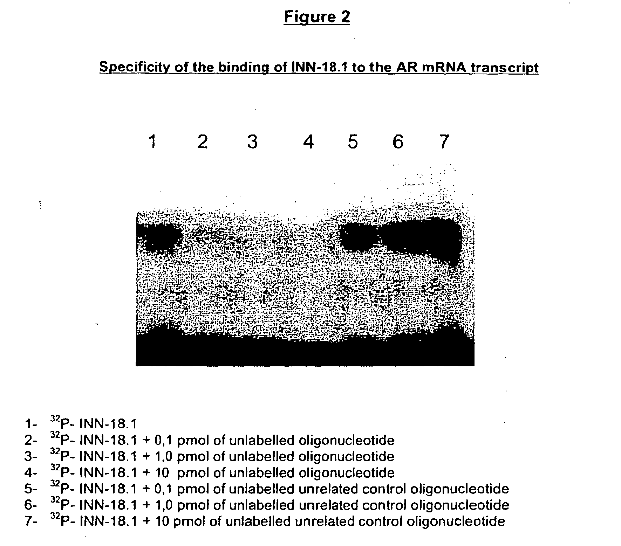

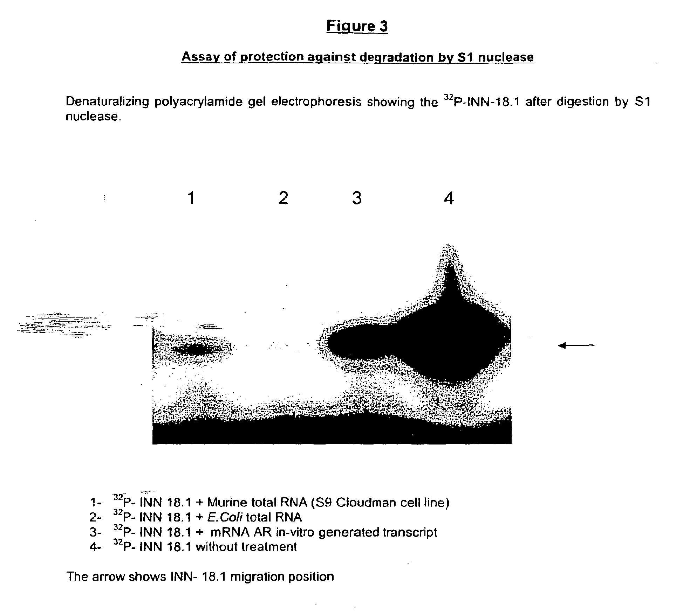

INN-18.1 binds specifically to the AR mRNA (figures 1 and 2), thus being completed the hybridization of the antiandrogen oligonucleotide (figure 3).

INN-18.1 hybridization to the AR mRNA induces an RNAse H digestion of the transcript (figure 4). Thus, the AR mRNA in vitro translation is strongly diminished (figure 5).

By virtue of the strategy known as "walking on the mRNA" and the initial following up that permitted to establish the INN-18.1 oligonucleotide, it was possible to design a new oligonucleotide acting via a similar mechanism of binding to the mRNA, digestion by RNAse H and inhibition of the translation, which attacks the same accesibility regions disclosed: it happens to be the INN-18.2 oligonucleotide.

### II. Pharmacological activity

In the second part, that aims to disclose the pharmacological activity, the oligonucleotides positive in the cultures derived from hair and skin cells were tested. The capacity of these oligonucleotides to modulate and down regulate the expression of the AR in these cell models was assessed.

The oligonucleotides according to the present invention are specific antiandrogens in human cell cultures. As a consequence of their pharmacological activity, the translated AR expression is significantly diminished in primary cultures of skin fibroblasts and hair follicle dermal papilla cells..

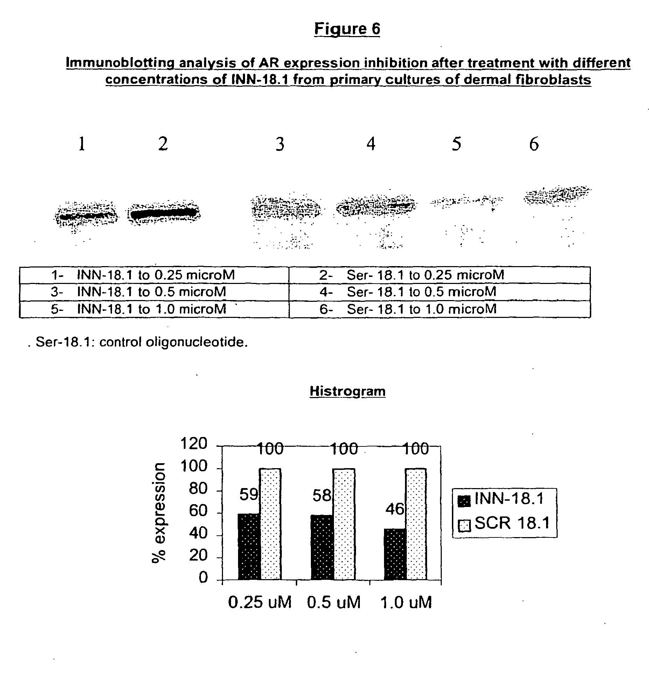

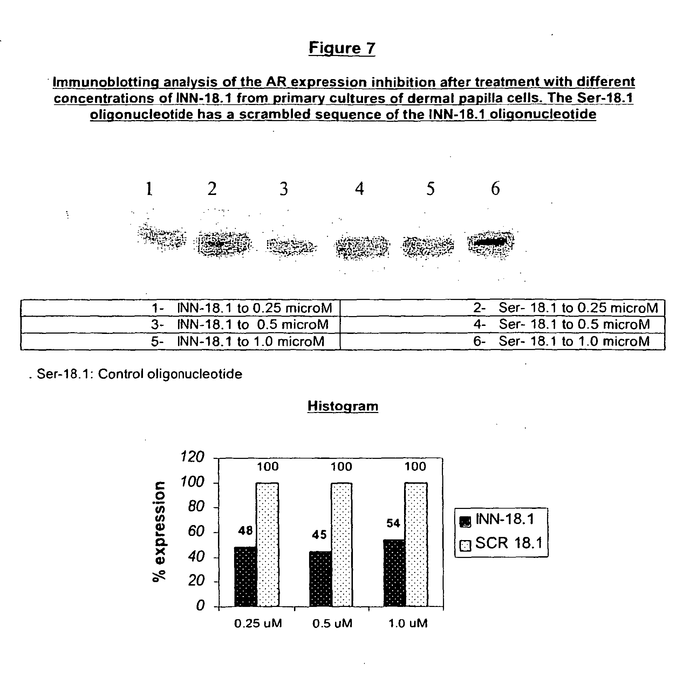

Antiandrogen activity of INN-18.1 induces the inhibition of the AR translation. Low level AR expression is detected by western blot in primary cultures of dermal fibroblasts (figure 6) and dermal papilla cells (figure 7).

In agreement with the disclosure of accessibility regions in the AR transcript molecule, and following the strategy called "mRNA walking", it was possible to establish the anti- androgen pharmacological activity of the new oligonucleotide INN-18.2 (figure 2), that induces the AR translation inhibition. Through western blot techniques low levels of AR expression in primary cultures of dermal papilla cells are detected.

The naked oligonucleotides, without protection by carriers or any pharmaceutical formulation especially created for such an end, have a very short half-life that is why, when they are administered topically, they reach their proximate tissue target in the skin, but not other, more distant, targets when administered systemically and, to attain a greater advantage, those oligonucleotides should be administered in the form of a water solution. As a consequence of their molecular composition, these anti-androgenic active principles are very specific for the androgen receptor, especially circumscribed to the site of application, namely in skin and scalp.

To sum up, due to the demonstrated inhibition capability in primary cultures of human skin fibroblasts and human hair follicle dermal papilla cells, these active principles are anti-androgens useful for the treatment of androgen - related dermatological pathologies.

### Main advantages over the previous art background

Because of their capability of triggering the cleaving of the mRNA through the RNAse H, these anti-androgen molecules are very specific, rendering a lower risk of toxicity or non-specific crossed secondary effects. The designed INN-18.1, INN-18.2 oligonucleotides are effective inhibitors of AR expression.

The present invention allows to disclose very sensitive accessibility regions (different and much more sensitive than the region which is the vicinity of the "near ATG" translation start) on the AR mRNA target, more suitable for the design of highly effective anti-androgen molecules.

The designed INN-18.1, INN-18.2 oligonucleotides are effective anti-androgens which can inhibit the AR expression in human skin fibroblasts and human hair follicle - derived dermal papilla cells, working at very low concentrations (250 nanoMM to 1 microM).

They target a more sensitive region of the AR mRNA: their inhibition effect could attain an inhibition greater than 60% - 80% at much lower concentrations (ranging from 1 microM to 0.25 microM), compared to the antisense oligomers targeting the "ATG near" region at concentrations higher than 10 microM and up to more than 40 microM.

In what follows, the invention is to be described by way of illustration, refering to the following experiments and embodiments, all of which are not to be deemed as limiting the invention.
Oligonucleotides marked with * are for reference only.

### Example 1. in vitro assesment of the designated active principles-electrophoretic mobility shift analysis (EMSA)

The electrophoretic mobility shift analysis comprises the following steps:

### a) Radio-labeling of oligonucleotides

The oligonucleotides were labeled by incubation at 37°C for 30 minutes at a final volume of 25 microL :
50 mM Tris-Cl pH 7.5
10 mM MgCl₂
5 mM DTT
10 pmol dephosphorylated oligonucleotides, at the 5' end
20 pmol (150 microCi) [gamma-³² P]ATP (specific activity >3000 Ci/mmol)
50 microg/ml BSA
3 U T4 polynucleotide kinase

The reaction was stopped by heating at 60°C for 5 minutes and an extraction was carried out with phenol/chloroform. Labelled oligonucleotides were separated from free ATP labelled by 20% polyacrylamide gel electrophoresis. Labelled oligonucleotides were identified by auto-radiography, sliced from gel and eluted at 37 °C during a whole night to a final volume of 120 microL of DEPC-treated water

### b) In Vitro Transcription of unlabeled Androgen Receptor (AR) mRNA

The AR cDNA was cloned in the pClneo expression plasmid (Promega) under the control of the T7 promoter. Before the in vitro transcription, the plasmid was linearized with Xba I (Promega).

The In vitro transcription of AR was carried out by incubation at 37°C for 90 minutes of the linearized (1 microg) in a 20 microL final volume ("In vitro transcription kit", Amersham-Pharmacia) containing 4 NTP (GTP, ATP, CTP, UTP) 0.5mM, DTT 5 mM, human placental ribonuclease inhibitor (HPR) 20 units/microL in 20 mM of HEPES-KOH, pH= 7.6, MgCl₂ 10 mM, T7 RNA Pol 20U. The reaction was stopped by heating to 75°C for 10 minutes.

Thereafter, 10 U of DNAse were added for 10 minutes at 37°C in order to cleave the template. The RNA transcript was precipitated in 2 volumes of ethanol, then kept overnight at -20°C, centrifuged 30 minutes at 12500 rpm at 4 °C. The resulting pellet was washed with ethanol 70% and, once re-centrifuged the pellet was dissolved in 20 microL of DEPC water.

### c)Hybridization of in vitro Androgen Receptor (AR) mRNA transcribed with radio-labeled oligonucleotides.

Radio-labeled oligonucleotides (1 fmol) were incubated with 50-100 fmol of AR mRNA at 37°C for 1h in a final volume of 20 microL containing 100 mM Na Cl, 10mM phosphate pH 7, 0.1 mM EDTA.

### d) Electrophoresis

The hybridization mixture was electrophoresed in a two-layer native polyacrylamide gel (4.5% - 20 %) for 4 hours at 4 °C in TBE 0.5X at 75V.

The following control reactions have been performed:
- Competition assay: hybridization in the presence of increasing amounts of the same unlabeled oligonucleotide.
- Competition assay: hybridization in the presence of a random sequence unlabeled oligonucleotide.
- Competition assay: hybridization in the presence of an unlabeled oligonucleotide corresponding to the "sense" sequence of the AR mRNA.
- Hybridization of the oligonucleotides with the anti-sense RNA transcript.

### Results: exploration of oligonucleotides through binding to the AR mRNA by EMSA

Figures 1, 8 and 15 (lane 2) show the electrophoretic mobility retardation, starting from the incubation of the radiolabeled oligonucleotides INN-18.1, INN-24* and INN-71* with the AR mRNA (sense AR transcript), the occurrence of the binding being indicated. The free oligonucleotides migrate faster at the front. No retardation could be seen when the same oligonucleotides incubated as control with another mRNA (in this case the "antisense" transcript) do not bind to it (lane 1).

In figures 2, 9 and 16, the specifity of this binding for the respective oligonucleotides is seen. Competition assays with increasing concentrations of the same unlabeled oligonucleotide were carried out, the disappearance of the retarded band because of an effect of isotopic dilution being seen (lanes 2 to 4 in figure). In the same way, competition assays of each nuecleotide with increasing amounts of an unlabeled irrelevant oligonucleotide (unrelated to the AR) have no effect on the retardation assay (lanes 5 - 7 in each figure), indicating that the electrophoretic mobility shift in lanes 2 to 4 is due to the specific binding to the AR transcript

### Example 2 S1 nuclease digestion protection assay

This technique indicates that both the ODN hybridize with the AR mRN preventing the oligonucleotide degradation, and the hybridization is complete or partial.

Partial hybridization (only a few nucleotides manage to hybridize with the target sequence) leads to a partial protection of the oligonucleotide molecule which, in turn, migrates faster in the gel electrophoresis. Complete hybridization (the whole oligonucleotide sequence hybridizes with the mRNA), results in a full - length oligonucleotide after the S1 digestion;

The S1 nuclease digestion protection assay consists of the following steps:

### a) Oligonucleotide radio-labeling and purification.

As described in item a) of the EMSA assay.

### b) In Vitro transcription of unlabeled Androgen Receptor (AR) mRNA

As described in item b) of the EMSA assay.

### c) Hybridization of in vitro transcribed Androgen Receptor (AR) mRNA with radio-labeled oligonucleotides

As described in item c) of the EMSA assay

### d) Nuclease S1 digestion

Thereafter, a Nuclease S1 digestion was carried out in a total volume of 20 microL for 1 hour at 37 °C. To the hybridization reaction, 2 microL of 10X Nuclease S1 buffer (30 mM sodium Acetate, pH= 4.6; 100 mM NaCl, 1 mM ZnSO₄) were added

### e) Electrophresis Casting of denaturing gel, electrophoresis and autoradiographs showing the size of the resulting oligonucleotide.

An aliquot (100,000 cpm) of the digestion mixture was electrophoresed a 20 % denaturing polyacrylamide gel.

### Results of the protection of oligonucleotides against digestion with S1 because of the hybridization to the AR mRNA

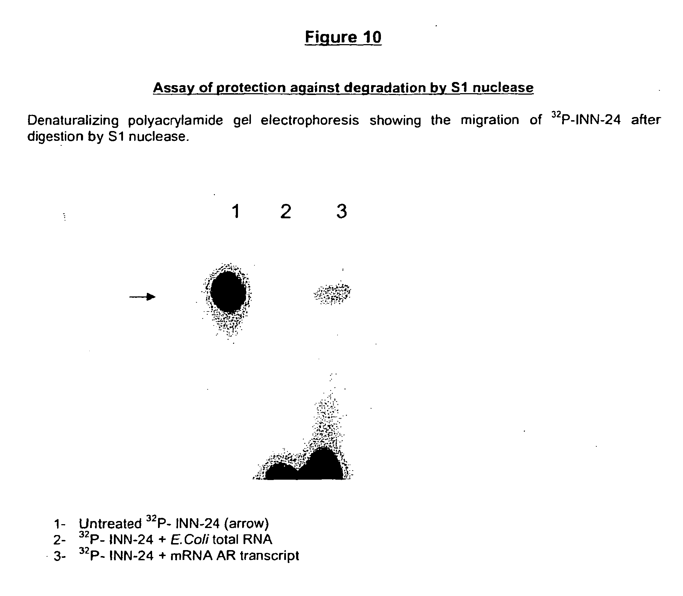

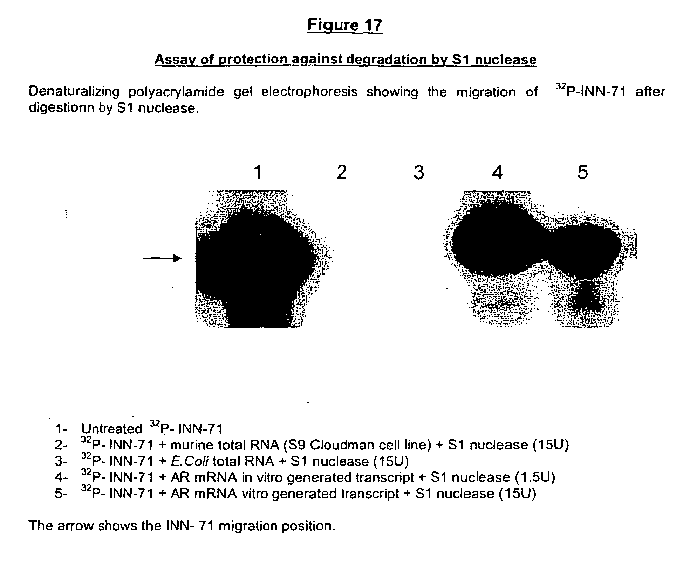

As it can be seen in figure 3, lane 3 (INN-18.1), figure 10 lane 3 (INN24*), figure 17 lanes 4 and 5 (INN-71*), oligonucleotides are protected against degradation through Nuclease S1 as a result from the hybridization to the AR mRNA. The hybridization rendering a double stranded heteroduplex, has a protection effect against the degradation by the nuclease S1, which is a single strand specific DNA nuclease. The oligonucleotides INN-18.1; INN-24*, INN-71* have kept their size (migrating in the same way their untreated oligonculeotide controls do), what indicates that the hybridization was complete. Negative controls were performed with irrelevant RNA from *E.coli* (figure 3 lane 2. figure 10 lane 2, figure 17 lane 3), with no signal being noted. When negative controls were performed with murine RNA, a weak signal could be seen for INN-18.1, because of the presence of a similar target sequence (figure 3, lane 1; figure 17, lane 2) .

### Example 3: RNA mRNA digestion by RNAse H

This technique can show that the hybridization capability of the ODN's could trigger an RNAse H mechanism of mRNA digestion which, within the cells, will be responsible for the androgen receptor messenger (AR-mRNA) degradation. This mechanism leads to the diminution of the AR expression, thus an anti-androgenic activity can be displayed. The RNAse H degradation involves the following steps:
a. Oligonucleotide radio-labeling and purification.
   As described in item a) of the EMSA assay
b. In vitro transcription of the androgen receptor (AR) mRNA
   as described in item b) of the EMSA assay
c. Nuclease S1 digestion
   The labeled AR mRNA transcript was incubated for 1 hour at 37°C with 1.25 microM of the oligodeoxynucleotide and 0.25 U/microL of RNAse H in a buffer containing 100 mM NaCl; 10 mM phosphate pH=7, 0.1mM EDTA and 1 mM MgCl₂. Digestions were carried out in a total volume of 10 microL.
d. Casting of denaturing gel, electrophoresis and autoradiography.
   The digestion product (100 000 cpm) was analyzed by agarose denaturing gel electrophoresis (0.7 % agarose, 20 mM MOPS (pH= 7)) , 8 mM sodium acetate, 1 mM EDTA pH= 8.0). The gel was then dried and exposed to film autoradiography.

### Results.

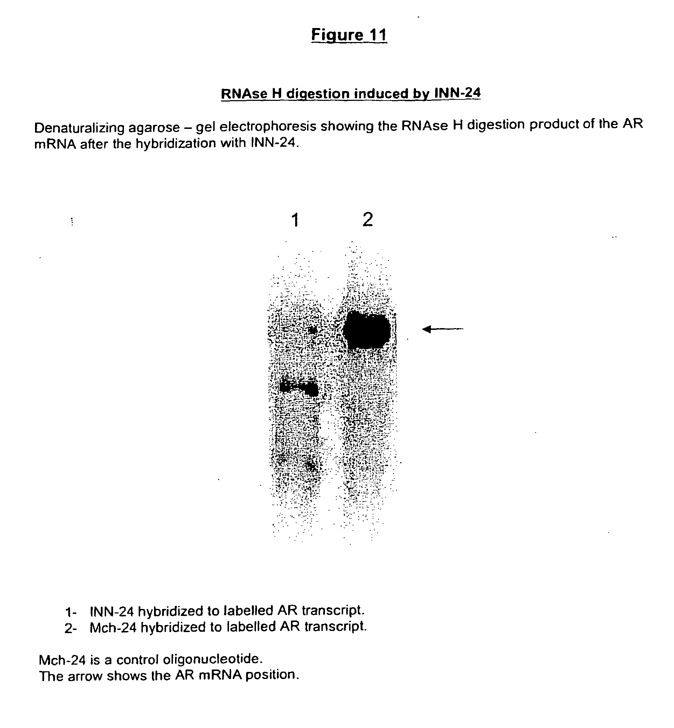

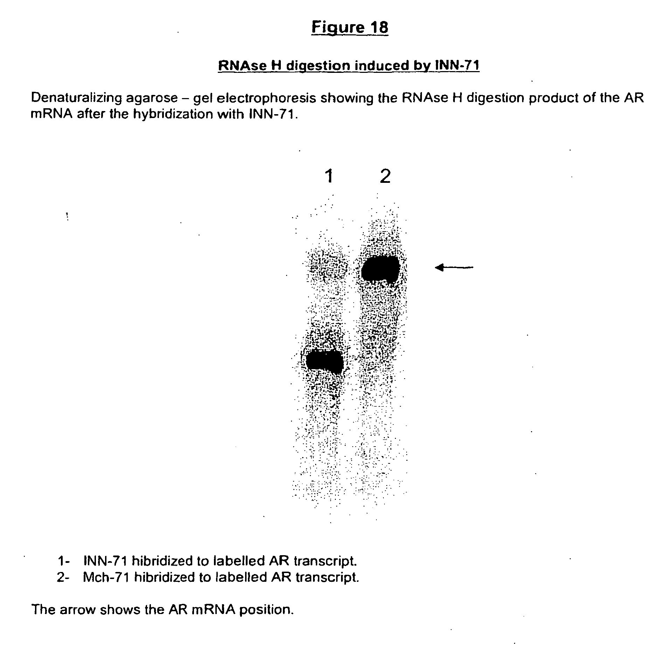

The INN-18.1 (figure 4 lane 1), INN-24 (figure 11 lane 1*) and INN-71 (figure 18 lane 1*) allow the RNA degradation through the RNAse H digestion. As a result of the degradation in the region where the heteroduplex is formed, the transcript is cut in two parts that migrate separately according to their length. The oligonucleotide acting as negative control, and which has the same sequence as the tested oligonucleotide's, save for three bases that were replaced (Mch-X), can be seen in lane 2 of all the figures, with no degradation promoting activity.

Figure 4 lane 1 shows two smaller RNA bands (about 3000 nt and, in the front, a weak one about 1000 nt).

Figure 11 lane 1 shows two smaller RNA bands about 2900 nt and 1400 nt.

Figure 18 lane 1 shows a faster thick band, probably consisting of two unresolved fragments of 2400 nt and 1900 nt.

The intact RNA can that can be seen in each figure is probably due to an non complete RNAse H activity.

### Example 4: In vitro translation inhibition.

This technique reinforces the information given by the use of RNAse H digestion. It is shown that the inhibition can be achieved in conditions of translation of the protein. Hereunder two alternative procedures which have been followed are described.

One-step procedure:
a. In vitro transcription of Androgen Receptor (AR) mRNA.
b. Incubation of in vitro transcribed RNA with the unlabeled oligonucleotide.
c. Incubation with RNAse H and a rabbit reticulocyte lysate.
d. One-dimensional gel electrophoresis under denaturing conditions and autoradiography.

Two-step procedure:
a. In vitro transcription of Androgen Receptor (AR) mRNA.
b. Incubation of in vitro transcribed RNA with unlabeled oligonucleotide.
c. Incubation with RNAse H.
d. Translation with a rabbit reticulocyte lysate.
e. One-dimensional gel electrophoresis under denaturing conditions and autoradiography.

### One - step procedure

### Transcription /translation reaction of AR.

The reaction was performed with the TnT Coupled Reticulocyte Lysate Systems (Promega) at 30°C for 90 minutes. The reaction was carried out in a final volume of 50 microL 1 microg of linearized AR cDNA was incubated with 25 microL of TnT rabbit reticulocyte Lysate, 2 microL of TnT reaction buffer, 1 microL of TnT T7 RNA polymerase, 1 microL of 1 mM amino acid mixture minus methionine , 2 microL of (³⁵S)-Methionine (>1000 Ci/mmol at 10mCi/ml - Amersham Pharmacia) and 40 units of Rnasin Ribonuclease Inhibitor in the presence of the oligonucleotide to be tested at 1.25 microM or control conditions without oligonucleotide hybridization.

### POLYACRYLAMIDE - Gel ELECTROPHORESIS Analysis of Translation Products

An aliquot (10 microL) of the translation reaction was denatured in 40 microL of SDS loading buffer (50 mM Tris-HCl pH= 6.8, 2% SDS, 0.1% bromophenol blue, 10% glycerol, 100 mM dithiothreitol) and loaded onto a 10 % SDS-polyacrylamide gel. The gel was fixed for 30 minutes in a 10 % glacial acetic acid fixing solution -50% methanol. Following the fixation, the gel was dried and exposed to autoradiography.

### Two - step procedure

### In vitro transcription of Androgen Receptor (AR) mRNA

In vitro transcription of AR m RNA was carried out as described in the item 2 of the EMSA protocol. The AR mRNA transcript was incubated with 1.25 microM of the oligodeoxynucleotide and 0.25 U/microL of RNAse H, as described above in the example 3) about the RNAse H digestion.

### Translation reaction

The translation reaction was carried out by incubation of all the transcription product (AR m RNA) after the treatment with RNAse H, 4 microL of Translation Mix 12.5X minus methionine (25mM HEPES pH7.6, 100mM creatine phosphate and 19 aminoacids (312.5 microM each), 100 mM potassium acetate , 0.5 mM magnesium acetate, 20 microL of reticulocyte lysate (supplemented with calf liver tRNA, EGTA, creatine phsphokinase and hemin) and 4 microL of (³⁵S)-Methionine (>1000 Ci/mmol at 10mCi/ml - Amersham Pharmacia). The reaction was performed at 30°C for 90 min in a final volume of 50 microL.

### Analysis of translation products

5 microL of translation reaction were diluted with 50 microL of sample buffer (0.5 ml 1 M Tris-HCl pH 6.8, 0.8 ml glycerol, 1.6 ml 10% (w/v) SDS, 0.4 ml 2-mercatpethanol, 0.1 ml saturated Pyronin Y solution, 4.6 ml water). The proteins were boiled in a water bath for 4 minutes, to denature; a 10 microL aliquot was loaded onto the gel. The samples were separated by SDS-PAGE. the electrophoresis was carried out in 8% polyacrylamide mini-gel at 160 V for 1 h. Molecular markers were loaded onto one of the lanes. The gel was fixed in 7 % (v/v) acetic acid for 1 hour at room temperature and dried for 1 hour at 60°C using a gel dryer, to be later exposed to autoradiography.

### Results: In vitro translation inhibition of the AR mRNA

As a consequence of the transcript degradation by RNAse H activity, the translation of the AR is strongly inhibited (figures 5, 12 and 19 lane 2).

### Example 5: Assessment of anti-androgenic activity in cell models

This technique allows to establish suitable models to assess the molecules INN-18.1, and INN-18.2 that are presented in this invention.

These assays aim towards disclosing the pharmacological activity; the positive oligonucleotides existing in cultures derived from hair follicle and skin cells were tested. It was evaluated the capability of these oligonucleotides for the modulation and down regulation of the AR expression in these cell models.

### 1) Setting up of cell cultures.

Cell cultures may be:
A) Primary cultures of dermal papilla cells.
   - Isolation and culture.
B) Primary cultures of human skin fibroblasts.
   - Isolation and culture.

### A) Isolation and culture of dermal papillae.

Full depth skin samples were obtained as the by-products of normal surgical procedures. Non blading specimens were obtained from the occipital human scalp of individuals undergoing corrective surgery for the treatment of androgenetic alopecia. The fascia and part of the subcutaneous tissue from scalp were carefully removed to thus expose as many hair bulbs as possible. The samples were washed with PBS containing penicillin (100 U/ml), streptomycin (100mg/ml) and Fungizone (2mg/ml). They were cut into 0.2 x 0.5 CM² fragments, put into dispase solution (Grade II, 50 U/ml. Gibco) and incubated at 37°C for one hour. Thereafter, all surgical procedures were performed under a dissection microscope (Nikon SMZ1000). Each anagen follicle was dissected free from its surrounding tissue and cut tranversewise transected about 1 mm above its base. The hair bulb was transferred to a Petri dish filled with medium. The epithelium was separated from the fibrous sheath and dermal papilla by the application of gentle pressure exerted on the proximal end of the follicle with the rounded tip of a needle. The fibrous sheath was then incised proximally, using the bevelled edge of a needle, thus a slight pressure being achieved that made the dermal papilla emerge through the cut. The papilla was cut along its stalk and transferred to a culture vessel. The papilla explants were placed in 35 mm plastic Petri dishes, generally four to eight in each dish. Culture medium DMEM containing penicillin (100 U/ml), streptomycin (100 mg/ml) and supplemented with 20% foetal bovine serum was used. Once the culture was established, the medium was changed every third day. 48 hours after the attachment, the cells began to migrate from the explants towards the plastic substrates. Following the cell migration there was a delay of 1 to 3 weeks before proliferation activity became apparent. The cell cultures were maintained and established in a humidified atmosphere containing 5 % CO₂.

### b) Dermal skin fibroblasts culture.

Dermal fibroblasts were obtained from small pieces of dermis. Each piece was checked under the dissecting microscope to ensure the absence of epidermal or muscle derivatives. Ten to fifteen pieces were transferred to 100 mm culture dishes, the attachment without medium culture being allowed during 15 to 20 minutes. Explants were thus cultured in DMEM medium supplemented with 10% foetal bovine serum and 10⁻⁷ M dihydrotestosterone (DHT). The primary cultures were left undisturbed for 1 week prior to examination under a phase contrast microscope, this followed by medium changes every week. After 4 to 5, weeks when the quantity of cells was sufficient, they were sub-cultured to establish primary cell lines.

### 2) Detection of AR expression in primary cell cultures. Western Blot Analysis

The technique involves two steps:
a) Antiandrogenic-oligonucleotide inhibition assay. Primary Cell Culture Treatment.
b) Androgen Receptor Expression Assay. Polyacrylamide Gel Electrophoresis and Western Blot Analysis.

### a) Antiandrogenic-oligonucleotide inhibition assay. Primary Cell Culture Treatment.

Dermal fibroblasts and dermal papillae cells (DPC) from primary cell cultures were seeded at 50 % confluence in 100 mm culture dishes for 18 to 24hr before the oligonucleotide treatment. Cells were were permitted to grow in DMEM medium supplemented with 10 % foetal bovine serum and 10⁻⁷ M dihydrotestosterone (DHT). Immediately before treatment, cells were washed with PBS and supplemented with an addition of fresh serum-free culture medium. The oligonucleotides at the desired concentration were mixed with polyethhylenimine (PEI) (50 % w/v aqueous solution - SIGMA) in a 1:5 charge ratio; then, they were diluted in 100 ml of DMEM medium and vortexed (transfection mixture). After 10 min, the transfection mixture was added to the cells. After 24 hr incubation, the cells were scrapped off the flask and collected in PBS. The cells were concentrated by centrifugation and resuspended in lysis buffer (125 mM Tris, 2 % SDS, 5 % Triton X-100, 2 mM phenylmethylsulfonyl fluoride (PMSF), pH 6.8). Extracts were centrifuged again and aliquots of the supernatant were used for protein determination.

### B) Androgen Receptor Expression Assay. Polyacrylamide Gel Electrophoresis and Western Blot Analysis.

Fifty microg of total proteins were diluted in loading buffer (125 mM Tris, 4 % SDS, 20% glycerol, 5 % mercaptoethanol, 0.01 % bromophenol blue , pH= 6.8). Once boiled, the samples were separated of sodium dodecyl sulphate-polyacrylamide electrophoresis (SDS-PAGE). Electrophoresis was carried out in 8% polyacrylamide mini-gels at 160 V for 1 hour. Molecular markers were applied to one of the lanes. Proteins were electro-blotted onto nitrocellulose membranes (Hybond ECL, Amersham Pharmacia). The membranes were blocked with 5% non - skimmed powder milk and incubated for at least 1.5 hr with a polyclonal antibody against the amino terminus end of the androgen receptor (AR) of human origin (rabbit polyclonal AR N-20 sc-816, Santa Cruz Biotechnology, Santa Cruz, CA) at 0.4 mg /ml in PBS-Tween-20 0.1 %. Once washed with PBS, the membranes were incubated with a monoclonal antibody against the carboxyl-terminus end of human actin (mouse monoclonal IgG1 antibody - Actin C-2 sc-8432 , Santa Cruz Biotechnology, Santa Cruz ,CA) for 1 hour, washed again and developed using a chemiluminescence's method (Amersham Pharmacia).

The resulting autoradiography was analyzed by densitometry using the Personal Densitometer SI and the Image Quant image analyzer system

### (Molecular Dynamics)

### RESULTS: Antiandrogenic pharmacological activity

The inhibition of the AR expression is presented as results of western blot experiments (upper part).

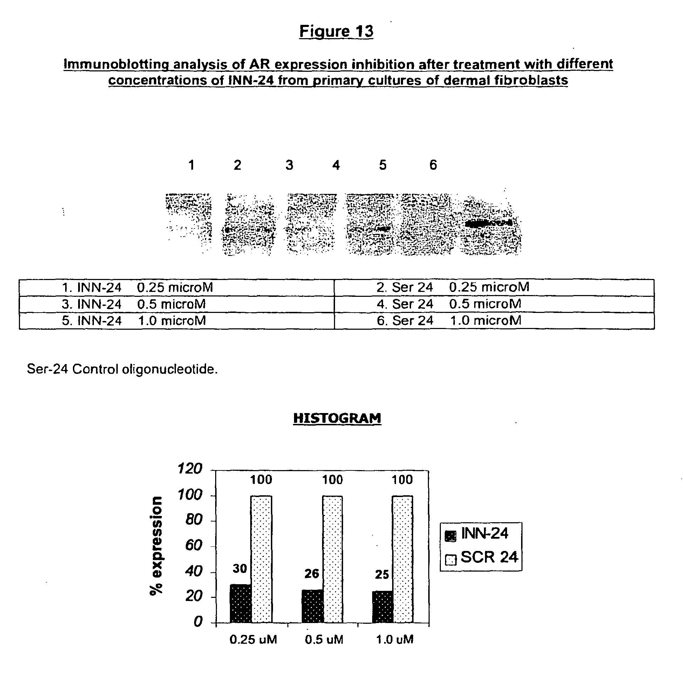

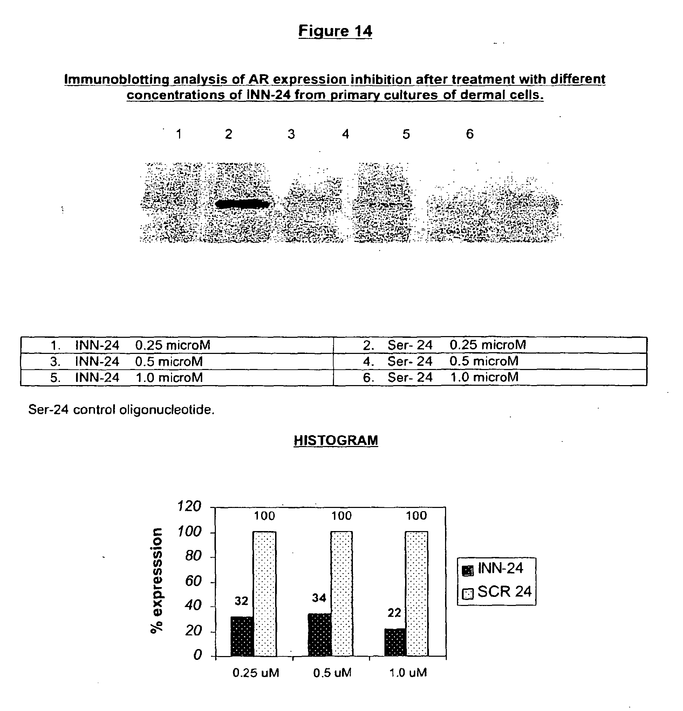

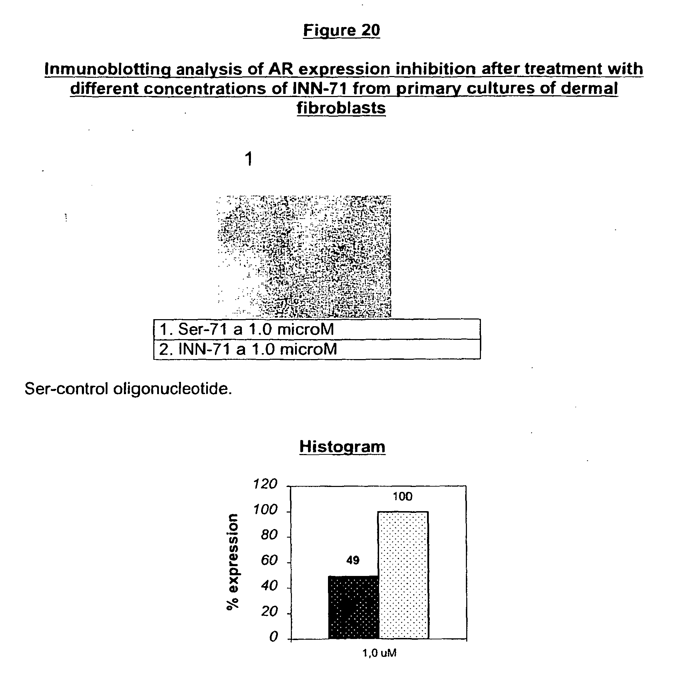

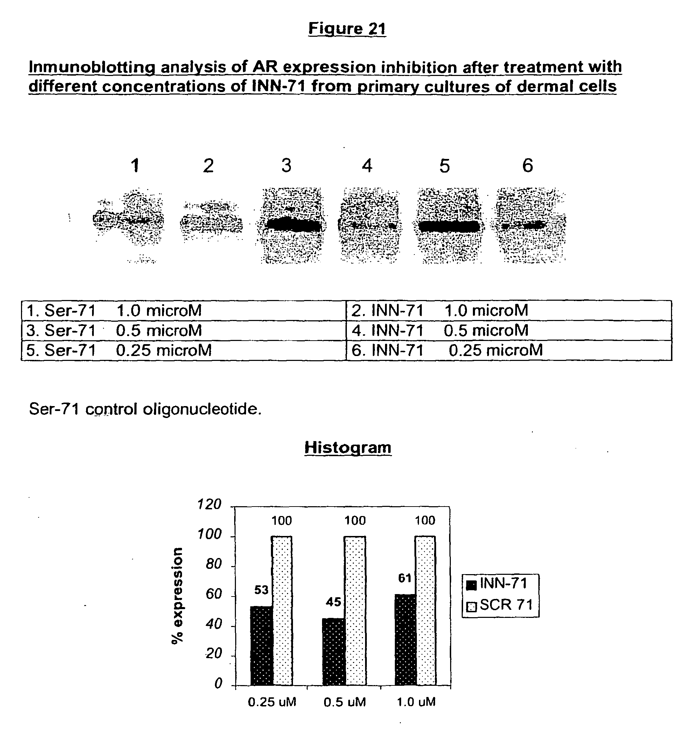

Figures 6, 13 and 20 correspond to primary cultures of skin fibroblasts and figures 7, 14 and 21, to dermal papillae primary cultures. The inhibition of the AR expression was assassed at different concentrations of INN-18.1, INN-24* and INN-71* are presented compared to non active controls , Scr-18.1, Scr-24* and Scr-71*, that have a mixed sequence of the respective oligonucleotide.

The histograms (lower figure) represent the percentage of expression relative to a control of non active oligonucleotides.

Each column's height represents a value relative to the Scr-x control, and that value is the mean of triplicate determinations carried out in independent experiments.

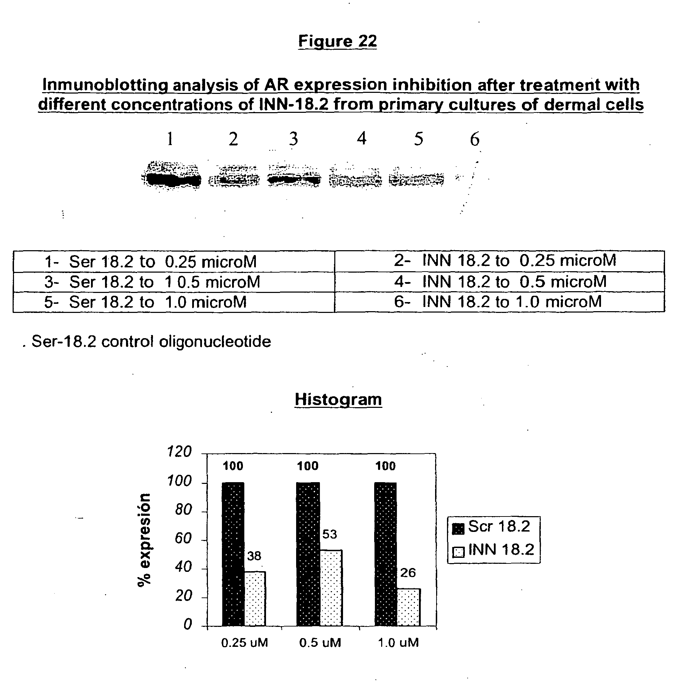

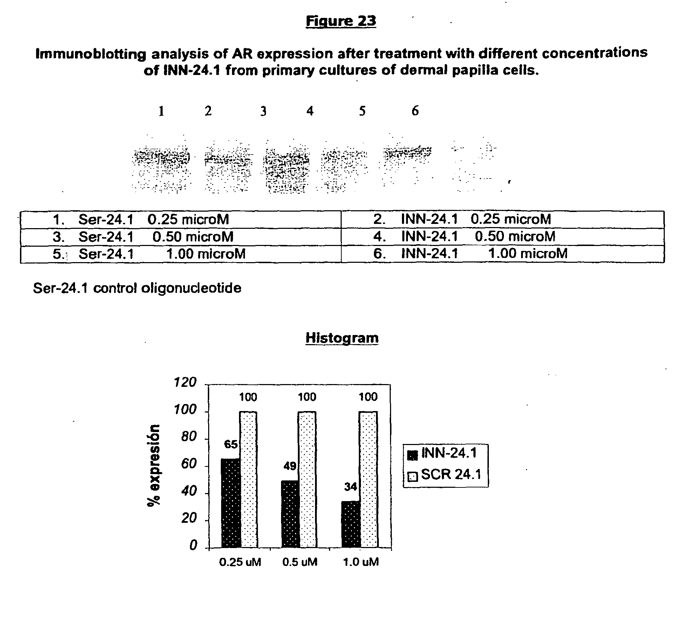

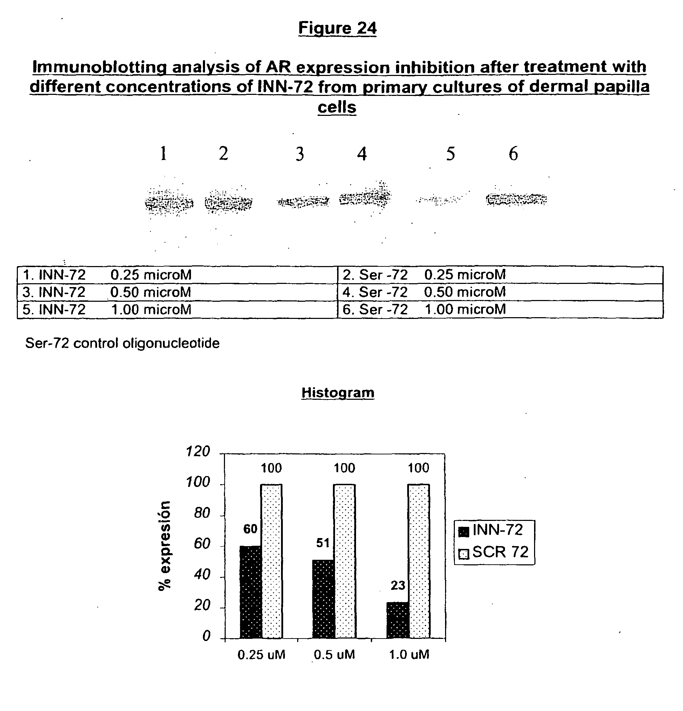

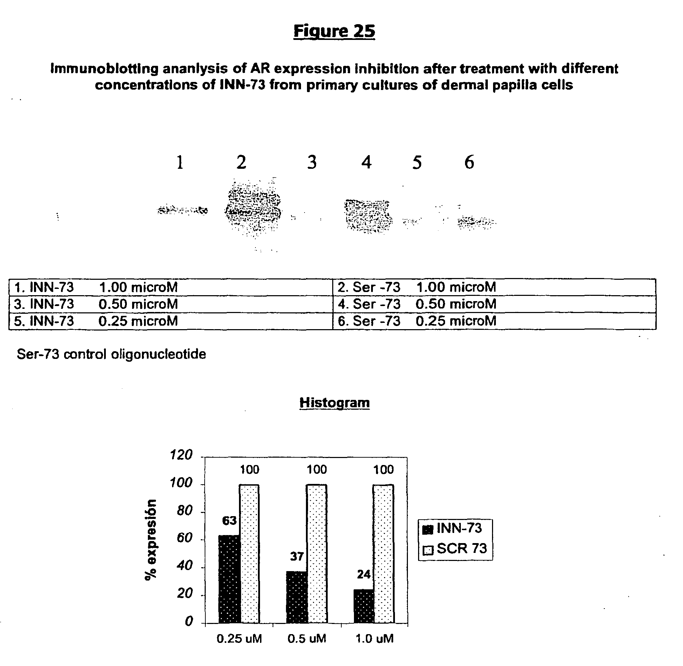

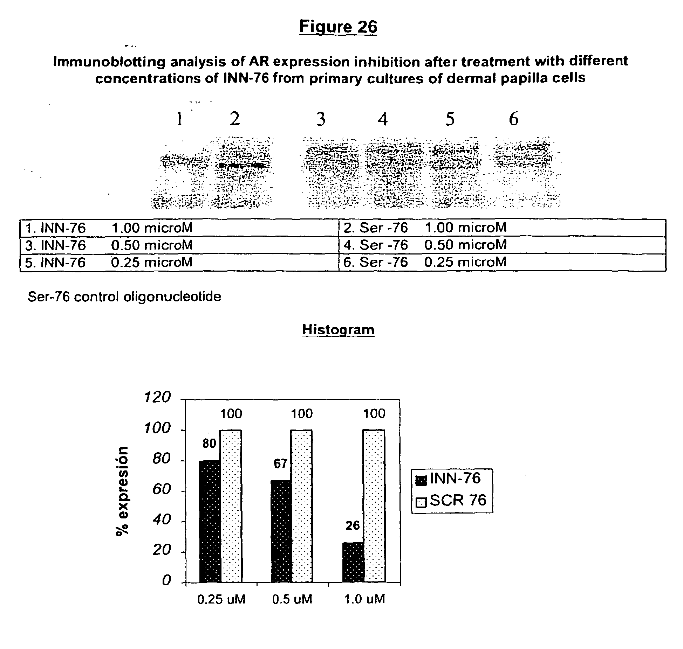

Following the same approach, the pharmalcological activity of primary dermal papilla cultures at different concentrations for INN-18.2 (figure 22), INN-24.1* (figure 23), INN-72* (figure 24), INN-73* (figure 25), INN-76* (figure 26) is shown in the corresponding Western blots.

## Claims

1. An antiandrogen oligonucleotide selected from the group consisting of SEQ ID N°: 1 and SEQ ID N°: 5.

2. An antiandrogen oligonucleotide according to claim 1 wherein SEQ ID NO: 1 and SEQ ID N°: 5 are DNA sequences, S-DNA sequences or DNA/S-DNA mixed sequences or alkylated or methylated derivates on the nitrogenous base.

3. A pharmaceutical and/or cosmetic composition **CHARACTERIZED in that** it contains an antiandrogen oligonucleotide according to claims 1-2 with a carrier or vector.

4. A composition according to claim 3 **CHARACTERIZED in that** it further contains pharmaceutically and/or cosmetically acceptable excipients and/or adjuvants.

5. A composition according to claim 3 **CHARACTERIZED by** being in a form suitable for topical administration.

6. A composition according to claim 3 **CHARACTERIZED by** being an aqueous solution.

7. The use of an antiandrogen oligonucleotide according to claims 1-2, **characterized in that** it is for the manufacture of a composition for the treatment of androgen-associated hair loss and androgen-skin related disorders.

8. The use according to claim 7 **CHARACTERIZED in that** said composition is in a form suitable for topical administration.

9. The use according to claim 8 **CHARACTERIZED in that** said composition is in the form of aqueous solution.

10. A method for treating androgen-associated hair loss in humans, **characterized in that** scalp cells are exposed to an effective amount of an antiandrogen oligonucleotides according to claims 1-2.

## Patentansprüche

1. Antiandrogen-Oligonukleotid, ausgewählt aus der Gruppe bestehend aus SEQ ID No: 1 und SEQ ID No: 5.

2. Antiandrogen-Oligonukleotid nach Anspruch 1, worin SEQ ID No: 1 und SEQ ID No: 5 DNS-Sequenzen, S-DNS-Sequenzen oder gemischte DNS/S-DNS-Sequenzen oder alkylierte oder methylierte Stickstoffbasenderivate sind.

3. Pharmazeutische und/oder kosmetische Zusammensetzung, **dadurch gekennzeichnet, dass** sie ein Antiandrogen-Oligonukleotid nach Anspruch 1 oder 2 mit einem Träger oder Vektor umfasst.

4. Zusammensetzung nach Anspruch 3, **dadurch gekennzeichnet, dass** sie ferner pharmazeutisch und/oder kosmetisch annehmbare Trägerstoffe und/oder Adjuvantien umfasst.

5. Zusammensetzung nach Anspruch 3, **dadurch gekennzeichnet, dass** sie in einer für die topische Anwendung geeigneten Form vorliegt.

6. Zusammensetzung nach Anspruch 3, **dadurch gekennzeichnet, dass** sie eine wässrige Lösung ist.

7. Verwendung eines Antiandrogen-Oligonukleotids nach Anspruch 1 oder 2, **dadurch gekennzeichnet, dass** es für die Herstellung einer Zusammensetzung für die Behandlung von androgenassoziiertem Haarausfall und androgenbedingten Hauterkrankungen ist.

8. Verwendung nach Anspruch 7, **dadurch gekennzeichnet, dass** die Zusammensetzung in einer für die topische Anwendung geeignete Form vorliegt.

9. Verwendung nach Anspruch 8, **dadurch gekennzeichnet, dass** sie in Form einer wässrigen Lösung vorliegt.

10. Verfahren zur Behandlung von androgenassoziiertem Haarausfall beim Menschen, **dadurch gekennzeichnet, dass** Kopfhautzellen einer wirksamen Menge eines Antiandrogen-Oligonukleotids nach Anspruch 1 oder 2 ausgesetzt werden.

## Revendications

1. Oligonucléotide anti-androgénique choisi dans le groupe comprenant SEQ ID N° : 1 et SEQ ID N° : 5.

2. Oligonucléotide anti-androgénique selon la revendication 1, dans lequel SEQ ID N° : 1 et SEQ ID N° : 5 sont des séquences ADN, des séquences S-ADN ou des séquences mixtes ADN/S-ADN ou des dérivés alkylés ou méthylés sur la base azotique.

3. Composition pharmaceutique et/ou cosmétique, **caractérisée en ce qu'**elle contient un oligonucléotide anti-androgénique selon les revendications 1 ou 2, avec un véhicule ou un vecteur.

4. Composition selon la revendication 3, **caractérisée en ce qu'**elle contient en outre des excipients et/ou adjuvants acceptables d'un point de vue pharmaceutique et/ou cosmétique.

5. Composition selon la revendication 3, **caractérisée en ce qu'**elle se présente sous une forme appropriée pour l'administration topique.

6. Composition selon la revendication 3, **caractérisée en ce qu'**elle est une solution aqueuse.

7. Utilisation d'un oligonucléotide anti-androgénique selon les revendications 1 ou 2, **caractérisée en ce qu'**il est destiné à la production d'une combinaison pour le traitement de la chute de cheveux associée aux androgènes et les troubles cutanés associés aux androgènes.

8. Utilisation selon la revendication 7, **caractérisée en ce que** ladite composition se présente sous une forme appropriée pour l'administration topique.

9. Utilisation selon la revendication 8, **caractérisée en ce que** ladite composition se présente sous la forme d'une solution aqueuse.

10. Procédé de traitement de la chute de cheveux associée aux androgènes chez l'être humain, **caractérisé en ce que** les cellules du cuir chevelu sont exposées à une quantité efficace d'oligonucléotides anti-androgéniques selon les revendications 1 ou 2.
